# EUROPÄISCHE PATENTSCHRIFT

(11) **EP 4 642 369 B1**
(45) Veröffentlichungstag und Bekanntmachung des Hinweises auf die Patenterteilung: **23.09.2026**
(21) Anmeldenummer: 24735955.7
(22) Anmeldetag: 20.06.2024
(51) Int. Cl.: A61B 34/20, A61B 34/00, A61B 34/30, A61B 90/00, A61B 90/50

(54) **CHIRURGISCHES STEUERSYSTEM, COMPUTERIMPLEMENTIERTES VERFAHREN ZUR STEUERUNG, SOWIE COMPUTERLESBARES SPEICHERMEDIUM**
SURGICAL CONTROL SYSTEM, COMPUTER-IMPLEMENTED CONTROL METHOD, AND COMPUTER-READABLE STORAGE MEDIUM
SYSTÈME DE COMMANDE CHIRURGICALE, PROCÉDÉ DE COMMANDE MIS EN OEUVRE PAR ORDINATEUR, ET SUPPORT DE STOCKAGE LISIBLE PAR ORDINATEUR

(30) Priorität: 22.06.2023 DE 102023116494
(43) Veröffentlichungstag der Anmeldung: 05.11.2025
(73) Patentinhaber: B. Braun New Ventures GmbH, 79110 Freiburg im Breisgau (DE)
(72) Erfinder: BEYL, Tim, 79115 Freiburg im Breisgau (DE); SARVESTANI, Amir, 79104 Freiburg (DE); STAWIASKI, Jean, 79117 Freiburg (DE)
(74) Vertreter: Winter, Brandl - Partnerschaft mbB
(86) Internationale Anmeldenummer: PCT/EP2024/067287
(87) Internationale Veröffentlichungsnummer: WO 2024/261149

(56) Entgegenhaltungen:
- EP-A1- 3 936 080
- WO-A1-2023/014732
- US-A1- 2007 265 495
- US-A1- 2019 223 964
- US-A1- 2021 169 605

## Beschreibung

### Technisches Gebiet

Die vorliegende Offenbarung betrifft ein chirurgisches Steuersystem zur Steuerung einer robotergeführten Visualisierungseinheit bei einem chirurgischen Eingriff. Der Roboter hat hierfür eine Roboterbasis als lokalen Anbindungspunkt des Roboters und insbesondere als lokales, ortsfestes Koordinatensystem oder Referenzkoordinatensystem. An der Roboterbasis ist ein beweglicher bzw. aktiv bewegbarer Roboterarm mit zumindest einem Roboterarm-Segment angebunden, an dem eine Visualisierungseinheit, insbesondere mit einer oder mehreren Kameras oder Ultraschall-Sonden, insbesondere an einer endständigen Seite des Roboterarms, angebunden, insbesondere gelagert ist. Die Visualisierungseinheit ist angepasst, eine insbesondere zeitaktuelle, korporale, vorzugsweise intrakorporale, Aufnahme des Patienten zu erstellen und, vorzugsweise digital, bereitzustellen. Daneben betrifft die vorliegende Offenbarung ein computerimplementiertes Verfahren zur Steuerung der Bewegung der Visualisierungseinheit, sowie ein computerlesbares Speichermedium und/oder ein Computerprogramm.

### Technischer Hintergrund

Im Bereich der Medizin bzw. Medizintechnik gewinnt eine Automatisierung mit einhergehender Einbindung digital steuerbarer technischer Vorrichtungen weiter an Bedeutung. Bei einem chirurgischen Eingriff werden vermehrt Roboter eingesetzt, welche insbesondere einen präzisen minimalinvasiven Eingriff unterstützen. Hierbei wird der Roboter nicht nur als alleinstehender Roboter vorgesehen, der einzig die Operation durchführt, sondern der Roboter wird verstärkt als kollaborativer Roboter (Cobot), also als assistierender bzw. unterstützender Roboter, direkt im Eingriffsfeld eingesetzt, welcher mit einem medizinischen Fachpersonal, insbesondere dem Chirurgen, interagiert.

Zu solch roboterunterstützen, chirurgischen Eingriffen, insbesondere neurochirurgischen Eingriffen am Gehirn, werden in der Regel verschiedene Visualisierungseinheiten zur physiologischen und funktionellen Beurteilung des Eingriffsgebietes und als Entscheidungshilfe eingesetzt. Zu den klassischen Daten, die dazu während des Eingriffs erfasst werden, gehören die Aufnahmen einer im Eingriffsgebiet platzierten Visualisierungseinheit, beispielsweise eines chirurgischen Mikroskops, Endoskops und/oder Exoskops und/oder einer Ultraschallsonde. Um dem Operateur die erforderliche Sicht zur Durchführung der Operation zu ermöglichen, muss die Visualisierungseinheit, genauer gesagt ein Kopf der Visualisierungseinheit mit entsprechender Optik oder entsprechendem Ultraschallwandler, laufend/ kontinuierlich neu positioniert werden.

Bei herkömmlichen Steuersystemen erfolgt dies beispielsweise mittels eines Joysticks. Bei dieser Lösung muss der Chirurg jedoch die Operation unterbrechen, indem er chirurgische Instrumente aus mindestens einer Hand nimmt, um den Joystick zu greifen und die Visualisierungseinheit neu zu positionieren. Herkömmliche, handfreie Lösungen verhindern demgegenüber die Unterbrechung der Operation, was Zeit spart und die chirurgische Konzentration erhöht. Bestehende Freihandlösungen sind beispielsweise ein Fußschalter, ein Mundschalter oder eine Sprachsteuerung.

Fußschalter und Mundschalter sind vergleichsweise wenig intuitiv und feinmotorisch zu bedienen, was die kognitive Konzentration des Operateurs binden und ihn vom Operationsgeschehen ablenken kann. Die Bedienung des Fußschalters beeinflusst einen Stand des Operateurs, sodass ggf. eine ergonomische Körperhaltung aufgegeben werden muss. Die Sprachsteuerung erweist als vergleichsweise wenig robust und verhindert rechtzeitige Bewegungen und auch sie beeinträchtigt die kognitive Konzentration.

Weitere Lösungen zur Steuerung eines Roboters bzw. einer Kamera sind insbesondere aus der US 2019/223964 A1, der WO 2023/014732 A1 der US 2007/0265495 A1 oder der US 2021/169605 A1 bekannt. Dabei werden Kopfbewegungen eines Bedieners erfasst und als Steuersignal verwendet.

### Zusammenfassung der vorliegenden Offenbarung

Es ist demgegenüber die Aufgabe der vorliegenden Offenbarung, Nachteile aus dem Stand der Technik zu vermeiden oder zumindest zu mindern und insbesondere ein chirurgisches Steuersystem, ein computerimplementiertes Verfahren zur Steuerung und ein computerlesbares Speichermedium und/oder Computerprogramm zur Verfügung stellen, welches eine besonders gute und intuitive Steuerung eines Visualisierungssystems bereitstellt. Eine Teilaufgabe kann darin gesehen werden, eine handfreie Steuerung bereitzustellen, bei der der Chirurg weiterhin beide Hände für seinen Eingriff nutzen kann, etwa ein Instrument halten kann, jedoch mit seinen Kopfbewegungen zusätzlich die Bewegung der Visualisierungseinheit und damit das Sichtfeld der Aufnahme steuern kann. Eine Teilaufgabe kann auch darin gesehen werden, mit möglichst intuitiv einsetzbaren Mitteln die Steuerung des Roboters für die Visualisierung bei einem Eingriff durchzuführen, wobei diese Mittel handfrei geführt werden und eine hohe Ergonomie aufweisen.

Die Aufgaben werden hinsichtlich eines chirurgischen Steuersystems erfindungsgemäß durch die Merkmale des Anspruchs 1 gelöst sowie hinsichtlich eines Verfahrens durch die Merkmale des Anspruchs 14 bzw. hinsichtlich eines computerlesbaren Speichermediums und/oder Computerprogramms durch die Merkmale des Anspruchs 15 gelöst. Vorteilhafte Weiterbildungen sind Gegenstand der Unteransprüche.

Ein Grundgedanke der Offenbarung liegt also darin, ein chirurgisches Steuersystem zu schaffen, das eine robotergeführte Visualisierungseinheit hat, die insbesondere ein chirurgisches Mikroskop oder Endoskop oder Exoskop oder eine chirurgische Ultraschallsonde - oder eine Auswahl davon - umfasst, und das angepasst ist, die Bewegung der Visualisierungseinheit mittels einem Kopf-Trackingsystem, also mittels einem (räumlichen) Kopf-(Nach-)Verfolgungssystem, welches die Kopfbewegung des Operateurs trackt oder (nach-)verfolgt, zu steuern. Diese Form der Steuerung ist höchst intuitiv und bringt den Vorteil mit sich, dass die Hände für die operative Tätigkeit frei bleiben. In anderen Worten wird die Bewegung der Visualisierungseinheit, und damit deren Sichtfeld und optische Achse, welche die Aufnahme definieren, relativ zu Kopfbewegungen des Operateurs gesteuert. Diese Steuerung oder Nachführung kann kontinuierlich/ permanent aktiv sein und/oder sie ist durch den Operateur punktuell, insbesondere bei Bedarf, aktivierbar. Das Trackingsystem kann auf einem bildbasierten Tracking des Kopfes des Operateurs basieren, sodass das Tracking ohne zusätzliche Marker auskommen kann und stattdessen beispielsweise das Gesicht des Operateurs verfolgt wird und auf eine Kopfbewegung hin analysiert wird. Ergänzend oder alternativ kann das Trackingsystem auf einem markerbasierten Tracking des Kopfes basieren. In diesem Falle bietet es sich an, dass ein Navigationstracker an einer ohnehin am Kopf des Operateurs vorgesehenen Vorrichtung angeordnet ist. Als Vorrichtung zu nennen ist hier insbesondere eine Operationsbrille, die von dem Operateur während eines Eingriffs, vorzugsweise permanent, getragen wird. Die Operationsbrille ist vorzugsweise eine passive, insbesondere auf Polarisation basierende, 3D-Brille, durch die bei Betrachtung einer externen, auf 3D-Technologie basierenden Anzeigevorrichtung, insbesondere eines Monitors oder Bildschirms, eine 3D-Anicht ermöglicht ist. Alternativ ist die Operationsbrille als eine aktive Virtual Reality- bzw. VR-Brille ausgestaltet und beinhaltet somit ein Anzeigesystem, insbesondere einen Monitor bzw. Bildschirm.

Konkret weist ein chirurgisches Steuersystem, insbesondere ein neurochirurgisches Steuersystem, einen, insbesondere navigierten, chirurgischen Roboter zur Verwendung bei einem chirurgischen Eingriff auf. Der Roboter hat eine Roboterbasis als lokalen Anbindungspunkt des Roboters und einen an die Roboterbasis angebundenen, bewegbaren Roboterarm mit einer Vielzahl an Roboterarm-Segmenten, welche mittels Gelenken miteinander verbunden sind und eine aktive Konfiguration, das heißt Pose oder Haltung, des Roboterarms ermöglichen. Am Roboterarm, insbesondere an einer endständigen Seite des Roboterarms, ist eine Visualisierungseinheit (oder ein Visualisierungssystem) als Endeffektor angebunden, insbesondere gelagert. Die Visualisierungseinheit ist angepasst, zumindest eine, insbesondere intrakorporale, Aufnahme des Patienten, insbesondere gemäß ihrer optischen Achse und ihres Sichtfeldes, zu erstellen und in Folge digital und somit computerlesbar bereitzustellen. Die Visualisierungseinheit hat insbesondere einen Mikroskopkopf eines Operationsmikroskops oder einen Visualisierungskopf eines Operationsendoskops oder -exoskops oder einer Ultraschallsonde, oder eine Auswahl solcher Köpfe. Des Weiteren hat das Steuersystem offenbarungsgemäß und abweichend von herkömmlichen Steuersystemen ein Kopf-Trackingsystem, das dafür angepasst ist, zumindest eine Kopfbewegung eines Operateurs zu erfassen. Zudem hat das Steuersystem eine Steuereinheit. Die Steuereinheit ist vorzugsweise und abweichend von herkömmlichen Steuersystemen dafür angepasst, die erfasste Kopfbewegung, insbesondere als ein Eingangssignal, in, insbesondere translatorische und/oder rotatorische, Bewegungskomponenten in oder um Achsen eines Koordinatensystems, insbesondere eines kartesischen Koordinatensystems, insbesondere des Roboters oder der Visualisierungseinheit, aufzugliedern. Die Steuereinheit ist vorzugsweise und abweichend von herkömmlichen Steuersystemen dafür angepasst, auf Basis der Bewegungskomponenten, unter Verwendung wenigstens einer in der Steuereinheit, insbesondere zur zumindest zeitweisen Ausführung, gespeicherten Steuervorschrift, ein Steuersignal für eine Bewegung der Visualisierungseinheit zu bestimmen und den Roboter mit diesem Steuersignal anzusteuern, sodass die Bewegung der Visualisierungseinheit an der Kopfbewegung des Operateurs orientiert und gemäß der Steuervorschrift robotergeführt erfolgt. Offenbarungsgemäß ist die Steuereinheit dafür angepasst, den Roboter mit einem in Abhängigkeit der erfassten Kopfbewegung bestimmten Steuersignal anzusteuern, sodass die robotergeführte Bewegung der Visualisierungseinheit in Abhängigkeit der erfassten Kopfbewegung erfolgt. Zudem hat das Steuersystem eine mit der Steuereinheit signalverbundene, manuell, insbesondere vom Operateur, betätigbare Aktivierungseinrichtung, die dafür angepasst ist, eine Steuerung der robotergeführten Bewegung der Visualisierungseinheit zu aktivieren oder zu deaktivieren. Dabei ist die Aktivierungseinrichtung als Totmannschalter ausgebildet ist, sodass die Steuerung der robotergeführten Bewegung der Visualisierungseinheit nur bei manuell, insbesondere vom Operateur, betätigter Aktivierungseinrichtung möglich ist.

So ist ein chirurgisches Steuersystem geschaffen, mit dem eine handfreie und besonders gute und intuitive Bewegungssteuerung einer Visualisierungseinheit bereitgestellt wird. Der Vorteil ist auch darin begründet, dass es kaum ein direkteres, und damit kaum ein intuitiveres, Mittel zur Bewegung eines Sichtfeldes der Visualisierungseinheit gibt, als die Kopfbewegung desjenigen, der das Sichtfeld der Visualisierungseinheit verändern möchte.

Gemäß einer Weiterbildung hat das chirurgische Steuersystem ein mit der Steuereinheit signalverbundenes Anforderungssystem, insbesondere ein Fußpedal und/oder einen Taster und/oder ein Mikrofon und/oder einen optischen Sensor, das dafür angepasst ist, eine Anforderung zur Aktivierung und/oder zu Deaktivierung der wenigstens einen Steuervorschrift zu erfassen, insbesondere eine Betätigung, einen Laut, eine Geste oder eine Mimik des Operateurs, und die Steuereinheit gemäß der Anforderung anzusteuern, die wenigstens eine Steuervorschrift zu aktivieren oder zu deaktivieren. Auf diese Weise ist ein Trigger gegeben, über den die Steuerung der Bewegung der Visualisierungseinheit durch die oben genannte Kopfverfolgung aktiviert und/oder deaktivier werden kann. Alternativ kann die Aktivierung und Deaktivierung auch durch eine spezielle Kopfgeste oder durch ein System erfolgen, das automatisch entscheidet, wann der Operateur die Visualisierungseinheit bewegen möchte.

Grundlegend bietet die in der Steuereinheit vorgesehene, wenigstens eine Steuervorschrift die Möglichkeit, einer beliebigen Bewegungskomponente der Kopfbewegung eine beliebige Bewegungskomponente der Bewegung der Visualisierungseinheit zuzuweisen, diese Bewegungskomponente der Bewegung der Visualisierungseinheit zu skalieren oder sie sogar zu sperren.

Mit Vorteil sind in einer Weiterbildung mehrere unterschiedliche Steuervorschriften vorgesehen, um dem Operateur die Steuerung der Visualisierungseinheit mit Hilfe seiner Kopfbewegungen auf eine Weise zu ermöglichen, die an seinen individuellen Anforderungen und Präferenzen orientiert erfolgt.

Gemäß einer bevorzugten Weiterbildung sind daher in der Steuereinheit unterschiedliche, insbesondere vorbestimmte oder vordefinierte, Steuervorschriften zur Ausführung gespeichert.

Um eine gewünschte der Steuervorschriften als gültig auswählen zu können und/oder wechseln zu können, weist das Steuersystem in einer Weiterbildung eine mit der Steuereinheit signalverbundene Auswahleinheit auf, insbesondere einen Taster oder einen Touchpad, mittels der der Operateur die Auswahl und/oder den Wechsel auslösen kann.

Gemäß einer bevorzugten Weiterbildung ist über eine Steuervorschrift das Steuersignal derart bestimmt, dass die robotergeführte Bewegung der Visualisierungseinheit auf eine orbitale, vorzugsweise sphärische, Bewegung beschränkt ist. Vorzugsweise ist dabei ein Fokuspunkt der Aufnahme fix und die Bewegung ist auf einen Orbit, vorzugsweise eine Sphäre, der (die) sich um den Fokuspunkt erstreckt, beschränkt.

Hierbei sind gemäß einer Weiterbildung die Steuervorschriften in Form eines Menüs gelistet, aus dem der Operateur die gewünschte Steuervorschrift auswählen kann.

Mögliche vordefinierte Steuervorschriften sind vorzugsweise derart definiert, dass:
- eine Transformation zwischen dem Kopf und der Visualisierungseinheit oder eine Bewegungstransformation zwischen der Kopfbewegung und der Bewegung der Visualisierungseinheit konstant oder starr ist;
- die Bewegung der Visualisierungseinheit zur Kopfbewegung proportional ist;
- die Bewegung der Visualisierungseinheit zur Kopfbewegung skaliert ist;
- die Bewegung der Visualisierungseinheit zur Kopfbewegung in oder um unterschiedliche der Achsen unterschiedlich skaliert ist;
- die Bewegung der Visualisierungseinheit auf eine Auswahl translatorischer und/oder rotatorischer Achsen beschränkt ist;
- ein Fokuspunkt der Aufnahme fix ist und die Bewegung der Visualisierungseinheit auf einen Orbit des Fokuspunktes oder eine Sphäre um den Fokuspunkt beschränkt ist;
- die Kopfbewegung, die in Sichtrichtung des Operateurs gerichtet ist, die Bewegung der Visualisierungseinheit in Richtung von deren optischer Achse bewirkt, und dass die Kopfbewegung, die entgegen der Sichtrichtung des Operateurs gerichtet ist, die Bewegung der Visualisierungseinheit in Richtung entgegen ihrer optischen Achse bewirkt.

Konkrete Beispiele von Steuervorschriften, die die Bewegung der Visualisierungseinheit teilweise in ihren Freiheitsgraden beschränken sind unter anderem:
- eine x-y-Bewegung der Visualisierungseinheit ohne Rotation, nur in einer Ebene senkrecht zu ihrer optischen Achse: Bewegt sich der Kopf nach links/rechts oder nach oben/unten, resultiert das alleine in einer Bewegung der Visualisierungseinheit in der Ebene senkrecht zur optischen Achse. Eine Bewegung des Kopfes nach vorne und hinten bleibt unberücksichtigt;
- eine x-y-z-Bewegung der Visualisierungseinheit entlang allen drei Raumrichtungen: Bewegt sich der Kopf nach links/rechts, oben/unten und nach vorne/hinten, resultiert das in einer Bewegung der Visualisierungseinheit in der Ebene senkrecht zur optischen Achse und zu einem Vergrößern/Verkleinern oder zu einem Bewegen auf den Patienten zu/vom Patienten weg (z);
- eine x-y-z-rx-ry-rz-Bewegung in allen 6 Freiheitsgraden (Translation und Rotation) zur Erreichung jeder beliebigen Position der Visualisierungseinheit;
- eine rx-ry-Bewegung in nur zwei Winkeln, wobei die Visualisierungseinheit auf den Fokuspunkt fixiert wird, was dazu führt, dass sie sich wie auf einer Kugel um den Fokuspunkt bewegen lässt;
- eine rx-ry-Bewegung in zwei Winkeln und in der Tiefe (z), was zu einer Bewegung auf einer Kugel um den Fokuspunkt führt und eine Vergrößerung/Verkleinerung oder eine Bewegung auf den Patienten zu/vom Patienten weg zulässt;
- eine Bewegung der Visualisierungseinheit derart, dass eine starre Transformation zwischen dem Kopf des Operateurs und dem Kopf der Visualisierungseinheit erfolgt, im Sinne einer virtuellen Halterung am Kopf des Operateurs.

Grundlegend sind natürlich Kombinationen der genannten Steuervorschriften oder von Anteilen der genannten Steuervorschriften möglich, sofern die Steuervorschriften oder Anteile, die kombiniert werden sollen, zueinander nicht in Widerspruch stehen.

Gemäß einer Weiterbildung ist die Steuereinheit mit einer Eingabeeinheit dafür angepasst, dass der Operateur die Kombination vorbestimmter Steuervorschriften vornehmen kann und/oder eine individuelle Steuervorschrift anhand seiner individuellen Präferenzen definieren kann.

Vorzugsweise erfolgt die Bewegung der Visualisierungseinheit relativ zu ihrer aktuellen Position, das heißt insbesondere relativ zu ihrer Position bei Aktivierung der Steuervorschrift.

Vorzugsweise ist eine mögliche Referenz für eine Richtung der Bewegung der Visualisierungseinheit eine aktuelle Sichtrichtung des Operateurs oder eine Orientierung der Visualisierungseinheit.

Insbesondere bei Verwendung eines Monitors als Anzeigesystem zum Anzeigen der Aufnahme ist eine mögliche Referenzachse eine Linie zwischen dem Operateur und dem Monitor oder eine Normale des Monitors, die durch eine starre Transformation umgewandelt ist, um eine Rotation zwischen der Sichtrichtung des Operateurs und der optischen Achse der Visualisierungseinheit zu berücksichtigen.

Gemäß einer möglichen Weiterbildung ist der wenigstens einen Steuervorschrift eine in der Steuereinheit zur Ausführung gespeicherte Geschwindigkeitsvorschrift zugeordnet, durch die insbesondere ein Geschwindigkeitsprofil der Bewegung der Visualisierungseinheit mit Bezug zur Kopfbewegung, mit oder ohne Totzeit, eingestellt ist. Vorzugsweise ist die Geschwindigkeitsvorschrift als Default vorbestimmt zugeordnet. Gemäß einer Weiterbildung sind unterschiedliche Geschwindigkeitsvorschriften in der Steuereinheit zur Ausführung gespeichert und, insbesondere durch die Auswahleinheit, frei auswählbar und/oder wechselbar. Eine mögliche Geschwindigkeitsvorschrift ist beispielsweise so definiert, dass die Geschwindigkeit der Bewegung der Visualisierungseinheit stets proportional zur Kopfbewegung ist, oder dass die Geschwindigkeit der Bewegung der Visualisierungseinheit eine Dämpfung aufweist. Die Geschwindigkeitsvorschrift kann insbesondere von der Beschleunigung der Kopfbewegung abhängig sein. Auf diese kann - mit dem Ziel einer guten Verfolgbarkeit der Aufnahmen und/oder mit dem Ziel eines zügigen und gut verfolgbaren Wechsels des Sichtfeldes - die Bewegung der Visualisierungseinheit in ihrer Geschwindigkeit, ihrer Beschleunigung und ihrem Bewegungsumfang gezielt, und insbesondere von der Geschwindigkeit in gewissem Maße entkoppelt, gesteuert werden.

Gemäß einer Weiterbildung ist die Steuereinheit dafür angepasst, die Visualisierungseinheit mit einem in Abhängigkeit der erfassten Kopfbewegung bestimmten Verstellsignal anzusteuern, sodass - ergänzend oder alternativ zur Bewegung der Visualisierungseinheit - eine Verstellung eines inneren optischen Parameters der Visualisierungseinheit in Abhängigkeit der Kopfbewegung erfolgt.

Gemäß einer Weiterbildung ist die Steuereinheit dafür angepasst, die Visualisierungseinheit mit einem in Abhängigkeit der erfassten Kopfbewegung bestimmten Verstellsignal anzusteuern, sodass eine innere Verstellung eines Bildausschnitts oder Zooms in Abhängigkeit der erfassten Kopfbewegung erfolgt.

Gemäß einer Weiterbildung ist die Steuereinheit dafür angepasst, die Visualisierungseinheit mit einem in Abhängigkeit der erfassten Kopfbewegung bestimmten Verstellsignal anzusteuern, sodass eine innere Verstellung eines Fokus oder Brennpunktes der Visualisierungseinheit erfolgt.

Gemäß einer Weiterbildung ist die Steuereinheit dafür angepasst, das jeweilige Verstellsignal so zu bestimmen, dass die erfasste Kopfbewegung, die in Sichtrichtung des Operateurs gerichtet ist, ein Heranzoomen bewirkt oder eine Verlagerung des Fokus in Blickrichtung, das heißt nach vorne, bewirkt, und dass die Kopfbewegung, die entgegen der Sichtrichtung des Operateurs gerichtet ist, ein Herauszoomen bewirkt oder eine Verlagerung des Fokus entgegen der Blickrichtung, das heißt nach hinten oder zum Operateur hin, bewirkt.

Gemäß einer Weiterbildung ist die Steuereinheit dafür angepasst, auf die Bewegungskomponenten wenigstens eine in der Steuereinheit gespeicherte Verstellvorschrift anzuwenden, um das Verstellsignal zu bestimmen.

Gemäß einer Weiterbildung ist die Steuereinheit dafür angepasst, auf Basis der Bewegungskomponenten, insbesondere der in und entgegen einer Sichtrichtung des Operateurs weisenden Bewegungskomponenten, und unter Verwendung wenigstens einer in der Steuereinheit zur Ausführung gespeicherten Verstellvorschrift, ein Verstellsignal für eine innere Verstellung eines inneren optischen Parameters der Visualisierungseinheit, insbesondere für die Verstellung eines Zooms und/oder eines Fokus der Visualisierungseinheit, zu bestimmen und die Visualisierungseinheit mit diesem Verstellsignal anzusteuern, sodass die innere Verstellung des inneren optischen Parameters, insbesondere die Verstellung des Zooms und/oder des Fokus, erfolgt. In anderen Worten kann die Steuereinheit als Submodul eine Verstelleinheit aufweisen, welche ein Fokussystem oder ein optisches System der Visualisierungseinheit steuert, und auf Basis der Verstellvorschrift wird eine Kopfbewegung in eine Verstellung des Fokus oder des Zooms übertragen.

Eine mögliche, vordefinierte Verstellvorschrift ist vorzugsweise derart definiert, dass die Kopfbewegung, die in Sichtrichtung des Operateurs gerichtet ist, die Verstellung des Zooms und/oder Fokus, vorzugsweise eine Vergrößerung (etwa in Sichtrichtung nach vorne) des Zooms und/oder eine Verlagerung des Fokus bzw. der Fokusebene nach vorne, bewirkt, und dass die Kopfbewegung, die entgegen der Sichtrichtung des Operateurs gerichtet ist, die entgegen gerichtete Verstellung des Zooms und/ oder des Fokus, vorzugsweise eine Verkleinerung (etwa in Sichtrichtung nach hinten) des Zooms und/ oder eine Verlagerung des Fokus bzw. der Fokusebene nach hinten bewirkt.

Die Verstellung des Zooms und/ oder Fokus in Abhängigkeit des Kopf-Trackings kann insbesondere in Analogie zur vorbeschriebenen Steuervorschrift kontinuierlich/ permanent aktiv sein oder punktuell, insbesondere bei Bedarf, aktivierbar/ deaktivierbar vorgesehen sein. Zur Aktivierung/ Deaktivierung der Verstellvorschrift hat das chirurgische Steuersystem gemäß einer Weiterbildung entweder ein eigenständiges, mit der Steuereinheit signalverbundenes Anforderungssystem, oder das Anforderungssystem, mit welchem die Aktivierung/ Deaktivierung der zuvor beschriebenen Steuervorschrift erfolgt, ist ebenso zur Aktivierung/ Deaktivierung der Verstellvorschrift ausgestaltet.

Das zuvor genannte Anforderungssystem kann gemäß der vorliegenden Offenbarung als eine Aktivierungseinrichtung verstanden werden.

Gemäß einer bevorzugten Weiterbildung hat das Steuersystem eine mit der Steuereinheit signalverbundene, manuell, insbesondere vom Operateur, betätigbare Aktivierungseinrichtung, die dafür angepasst ist, eine Steuerung der inneren Verstellung des Bildausschnitts oder Zooms der Visualisierungseinheit und/oder eine Steuerung der inneren Verstellung des Fokus oder Brennpunktes der Visualisierungseinheit zu aktivieren oder zu deaktivieren.

Gemäß einer Weiterbildung ist die Aktivierungseinrichtung als ein Fußpedal oder ein Mundschalter ausgestaltet.

Gemäß einer bevorzugten Weiterbildung weist das Kopf-Trackingsystem auf:
wenigstens einen am Kopf des Operateurs befestigbaren Marker und eine extern angeordnete optische Erfassungseinheit, die dafür angepasst ist, den wenigstens einen Marker optisch zu erfassen. Unter extern angeordnet wird insbesondere nicht am Kopf des Operateurs befestigt verstanden.

Gemäß einer bevorzugten Weiterbildung weist das Kopf-Trackingsystem auf:
wenigstens eine optische Erfassungseinheit, die dafür angepasst ist, die Kopfbewegung mittels maschinellem Sehen des Gesichtes des Operateurs zu erfassen.

Die wenigstens eine optische Erfassungseinheit ist vorzugsweise eine optische 3D-Tracking-Kamera. Alternativ oder ergänzend ist wenigstens eine optische Operateur-Kamera als optische Erfassungseinheit vorgesehen, die den Operateur und sein Handeln beim Eingriff erfasst.

Gemäß einer bevorzugten Weiterbildung hat das Steuersystem eine visuelle Anzeigevorrichtung zur Anzeige der mit der Visualisierungseinheit erstellten und bereitgestellten Aufnahme des Eingriffsgebietes, die bevorzugt als ein externer Monitor ausgestaltet ist. Unter extern angeordnet wird insbesondere nicht am Kopf des Operateurs befestigt verstanden.

Gemäß einer bevorzugten Weiterbildung ist die wenigstens eine optische Erfassungseinheit an dem externen Monitor angeordnet oder befestigt, vorzugsweise an einem Rand, vorzugsweise oberen Rand, des externen Monitors. Ein Vorteil hierbei ist, dass der Operateur sein Gesicht während des Eingriffs ohnehin dem Monitor zuwendet/ zuwenden muss. Zudem unterliegt die Sichtachse zwischen dem Operateur und dem externen Monitor einem nur geringen Risiko, blockiert zu werden. Das gleiche gilt dann auch für die Sichtachse zwischen der optischen Erfassungseinheit und dem am Kopf des Operateurs befestigbaren Marker, bzw. dem Kopf/ Gesicht des Operateurs. Das Risiko, dass das Tracking des Kopfes/ des Gesichtes durch Blockieren der Sichtachse gestört wird, ist somit gering.

Alternativ kann die visuelle Anzeigevorrichtung als ein am Kopf befestigbarer 3D-Monitor, insbesondere eine Virtual-Reality-Brille, ausgebildet sein.

Um die Erfassung der Bewegung des Kopfes noch prozesssicherer zu machen, weist das Trackingsystem gemäß einer möglichen Weiterbildung wenigstens eine am Kopf befestigbare Beschleunigungssensoreinheit auf, und/oder wenigstens einen externen Marker und wenigstens eine am Kopf befestigbare Erfassungseinheit auf, die dafür angepasst ist, den wenigstens einen externen Marker zu erfassen.

In anderen Worten kann das Trackingsystem eine, insbesondere mobile, Tragevorrichtung für einen Kopf aufweisen und diese Tragevorrichtung zumindest einen der folgenden Komponenten aufweisen:
- einen trägheitsbasierten Inertialsensor,
- ein geometrisches Referenzsystem als externen Marker, welche durch insbesondere eine Trackingkamera erfasst wird,
- eine Trackingkamera, welche einen Marker erfassen kann
- vorzugsweise eine Energiequelle, etwa in Form eines Akkus, um autark die Tragevorrichtung betreiben zu können.

Die Tragevorrichtung kann insbesondere von einer Operationsbrille oder ein Head-Mounted-Display gebildet sein, die von dem Operateur während eines Eingriffs, vorzugsweise permanent, getragen wird, und die insbesondere eine passive, insbesondere auf Polarisation basierende, 3D-Brille ist, durch die bei Betrachtung eines externen, auf 3D-Technologie basierenden Anzeigesystems, insbesondere eines Monitors oder Bildschirms, eine 3D-Anicht ermöglicht ist. Alternativ ist die Operationsbrille insbesondere von einer Virtual Reality bzw. VR-Brille des Operateurs gebildet, auf deren Monitor bzw. Bildschirm die Aufnahme der Visualisierungseinheit projiziert wird.

Die Trackingkamera kann kopfbasiert oder extern, beispielsweise auf einem Wagen oder einem Ständer, angeordnet sein.

Die Marker können extern oder kopfbasiert angeordnet sein. Die Marker können in Form eines optischen Musters ausgebildet sein, beispielsweise als QR-ähnlicher Code, wenn eine oder mehrere Trackingkameras im sichtbaren Spektrum Teil des Trackingsystems sind, oder sie sind als Infrarot-Reflektormarker oder als IR-LEDs ausgestaltet, wenn eine oder mehrere Trackingkameras im Infrarot Spektrum Teil des Trackingsystems sind.

Das externe Tracking des Kopfes kann alternativ ohne Marker erfolgen, indem der Kopf des Operateurs und dessen Kopfbewegung mit Hilfe von maschinellem Sehen getrackt wird. Im Falle eines 3D Anzeigesystems mit einem 3D Monitor und einer 3D-Brille können die Marker an der 3D-Brille befestigt sein.

Das Anzeigesystem kann ein 3D-Monitor, ein 2D-Monitor oder die bereits erwähnte VR- oder eine Augmented-Reality-Brille sein oder haben.

Gemäß der Offenbarung definiert der Begriff der Bewegung der Visualisierungseinheit eine Lagedifferenz zwischen einer Ausgangslage und einer Endlage der Visualisierungseinheit. Die Ausgangslage ist diejenige, die die Visualisierungseinheit bei der Aktivierung der Steuerung aufweist, die Endlage ist diejenige, die sie bei der Deaktivierung der Steuerung aufweist.

Der Begriff "Lage" umfasst sowohl eine Position als auch eine Orientierung. Insbesondere kann die Lage mittels sechs Koordinaten angegeben werden, drei Positionskoordinaten X, Y und Z sowie maximal drei Winkelkoordinaten für die Orientierung.

Eine Sichtrichtung des Operateurs ist offenbarungsgemäß durch die Lage seines Kopfes definiert und ist die Richtung, in welche die Vorderseite des Kopfes weist und in der somit das Sichtfeld des Operateurs liegt.

Eine Blickrichtung der Augen des Operateurs ist offenbarungsgemäß durch die Lage der Augen definiert und ist die Richtung, in die die Augen blicken. Die Blickrichtung weicht von der Sichtrichtung in einem Maß ab, in dem die Augen aus ihrer natürlichen Neutrallage ausgelenkt sind.

Gemäß der Offenbarung definiert der Begriff der Kopfbewegung eine Lagedifferenz des getrackten Kopfes zwischen einer Ausgangslage und einer Endlage des Kopfes. Die Ausgangslage ist diejenige, die der Kopf bei einer Aktivierung der Steuerung aufweist, die Endlage ist diejenige, die er bei einer Deaktivierung der Steuerung aufweist.

Gemäß einer Weiterbildung hat das Steuersystem, insbesondere eine kopfbasierte Tragevorrichtung, ein Augen-Trackingsystem, das dafür angepasst ist, eine Blickrichtung der Augen des Operateurs zu erfassen. Ergänzend ist die Steuereinheit dafür angepasst, auf Basis der erfassten Blickrichtung, insbesondere mittels maschinellem Sehen, innerhalb der Aufnahme einen Blickpunkt zu bestimmen und die Visualisierungseinheit anzusteuern, den Blickpunkt zu fokussieren oder scharf zu stellen.

Offenbarungsgemäß weist ein computerimplementiertes Verfahren zum Steuern einer Bewegung einer robotergeführten Visualisierungseinheit bei einem chirurgischen, insbesondere neurochirugischen, Eingriff, insbesondere für ein chirurgisches Steuersystem gemäß einem Aspekt der vorstehenden Beschreibung, folgende Schritte auf, die die handfreie Steuerung der Visualisierungseinheit ermöglichen:
Erstellen und Bereitstellen einer zeitaktuellen Aufnahme eines Eingriffsgebietes, durch die Visualisierungseinheit;
Anzeigen der bereitgestellten Aufnahme, durch eine visuelle Anzeigevorrichtung.
Erfassen einer Kopfbewegung eines Operateurs durch ein Kopf-Trackingsystem;
Vorzugsweise Aufgliedern der erfassten Kopfbewegung, insbesondere als ein Eingangssignal, in, insbesondere translatorische und/oder rotatorische, Bewegungskomponenten in oder um Achsen eines Koordinatensystems, insbesondere eines kartesischen Koordinatensystems, durch eine Steuereinheit;
Vorzugsweise Bestimmen eines Steuersignals für eine robotergeführte Bewegung der Visualisierungseinheit auf Basis der Bewegungskomponenten der Kopfbewegung, unter Verwendung wenigstens einer in der Steuereinheit zur Ausführung gespeicherten Steuervorschrift;
Vorzugsweise Ansteuern des Roboters mit dem Steuersignal, sodass die robotergeführte Bewegung der Visualisierungseinheit auf Basis der Kopfbewegung und der Steuervorschrift erfolgt;
Bestimmen eines Steuersignals für eine robotergeführte Bewegung der Visualisierungseinheit in Abhängigkeit der erfassten Kopfbewegung, durch eine Steuereinheit; und
Ansteuern des Roboters mit dem Steuersignal, sodass die robotergeführte Bewegung der Visualisierungseinheit in Abhängigkeit der erfassten Kopfbewegung erfolgt, durch die Steuereinheit.

Gemäß einer bevorzugten Weiterbildung hat das Verfahren zudem Schritte, die wenigstens einen inneren optischen Parameter der Visualisierungseinheit, insbesondere einen Zoom und/oder Fokus, auf Basis der Bewegungskomponenten der Kopfbewegung verstellen. Diese Schritte sind insbesondere:
Bestimmen eines Steuersignals für eine innere Verstellung eines inneren optischen Parameters der Visualisierungseinheit auf Basis der Bewegungskomponenten der Kopfbewegung unter Verwendung wenigstens einer in der Steuereinheit zur Ausführung gespeicherten Verstellvorschrift; und
Ansteuern der Visualisierungseinheit mit diesem Steuersignal, sodass eine innere Verstellung des Zooms und/ oder Fokus der Visualisierungseinheit auf Basis der Kopfbewegung und der Verstellvorschrift erfolgt.

Um das Sichtfeld und/oder die optische Achse der Visualisierungseinheit und/oder die Anzeige der Aufnahme und/oder den Zoom und/ oder Fokus tatsächlich nur bei Bedarf zu verändern/ zu verstellen, weist das Verfahren gemäß einer Weiterbildung Schritte auf:
Erfassen einer Anforderung zur Aktivierung der wenigstens einen Steuervorschrift und/oder Verstellvorschrift, durch wenigstens ein mit der Steuereinheit signalverbundenes Anforderungssystem bzw. wenigstens eine mit der Steuereinheit signalverbundene Aktivierungseinrichtung,
Ansteuern der Steuereinheit, die wenigstens eine, insbesondere gültige, Steuervorschrift und/ oder Verstellvorschrift zu aktivieren, durch das Anforderungssystem bzw. die Aktivierungseinrichtung, und
Aktivieren der wenigstens einen, insbesondere gültigen, Steuervorschrift und/oder Verstellvorschrift, durch die Steuereinheit.

So ist sichergestellt, dass bei nicht aktiver Steuervorschrift und/oder Verstellvorschrift, die Kopfbewegung keine Bewegung der Visualisierungseinheit und/oder keine Verstellung des Zooms und/oder Fokus der Visualisierungseinheit auslöst und die von der Aufnahme gezeigte Sicht auf den Patienten, zumindest bezüglich der optischen Achse und/oder des Zooms und/oder Fokus der Visualisierungseinheit, konstant und ruhig bleibt, wohingegen bei bewusst aktivierter Steuervorschrift und/oder Verstellvorschrift, die Bewegung und/oder der Zoom und/oder Fokus der Visualisierungseinheit der Kopfbewegung im Sinne des Operateurs folgt oder folgen. Ein weiterer Vorteil der Deaktivierung der Steuervorschrift und/ oder Verstellvorschrift ist natürlich, dass der Operateur seinen Kopf nach Deaktivierung wieder in eine ergonomische Position bringen kann.

Entsprechend weist eine Weiterbildung des Verfahrens natürlich wenigstens eine Möglichkeit auf, die aktive Steuervorschrift und/oder Verstellvorschrift wieder zu deaktivieren. Das wird so umgesetzt, dass die Steuervorschrift und/oder Verstellvorschrift nur so lange aktiviert ist, bis durch das Anforderungssystem bzw. die Aktivierungseinrichtung eine Anforderung zur Deaktivierung erfasst wird oder bis von der Steuereinheit ein Deaktivierungskriterium als erfüllt bestimmt wird.

Das Verfahren weist demgemäß insbesondere die Schritte auf:
Erfassen einer Anforderung zur Deaktivierung der wenigstens einen Steuervorschrift und/oder Verstellvorschrift, durch das Anforderungssystem bzw. die Aktivierungseinrichtung, oder
Bestimmen eines Deaktivierungskriteriums als erfüllt, durch die Steuereinheit,
und in Abhängigkeit der erfassten Anforderung zur Deaktivierung oder des als erfüllt bestimmten Deaktivierungskriteriums einen Schritt:
   Deaktivieren der Steuervorschrift und /oder Verstellvorschrift durch die Steuereinheit.

Um die gültige Steuervorschrift und/oder Verstellvorschrift wechseln und an die Bedürfnisse des Operateurs anpassen zu können, kann das Verfahren vorzugsweise die Schritte aufweisen:
Auswahl einer von mehreren Steuervorschriften und/oder Verstellvorschriften als gültige Steuervorschrift und/oder Verstellvorschrift, durch eine Auswahleinheit, die dafür angepasst ist, eine aus mehreren Steuervorschriften und /oder Verstellvorschriften als gültige auszuwählen und/oder eine aktuell gültige der Steuervorschriften und/oder Verstellvorschriften zu wechseln, oder
Bestimmen einer von mehreren Steuervorschriften und/oder Verstellvorschriften als gültige Steuervorschrift und/oder Verstellvorschrift, durch die Steuereinheit.

Hinsichtlich eines computerlesbaren Speichermediums oder eines Computerprogramms werden die Aufgaben (jeweils) dadurch gelöst, dass das Speichermedium bzw. Computerprogramm Befehle umfasst, die bei einer Ausführung durch den Computer diesen veranlassen, die Schritte des Verfahrens gemäß der vorliegenden Offenbarung auszuführen.

Jegliche Offenbarung im Zusammenhang mit dem chirurgischen Steuersystem gemäß der vorliegenden Offenbarung gilt für das Verfahren gemäß der vorliegenden Offenbarung, wie auch umgekehrt.

### Kurzbeschreibung der Figuren

Die Erfindung wird nachfolgend anhand bevorzugter Ausführungsformen mit Hilfe von Figuren näher erläutert. Es zeigen:
- Fig. 1: eine schematische Seitenansicht eines chirurgischen Steuersystems mit einer robotergeführten Visualisierungseinheit, einem Anzeigesystem und einem Kopf-Trackingsystem zur Bewegungssteuerung der Visualisierungseinheit, gemäß einer bevorzugten Ausführungsform;
- Fign. 2a bis 2d: Seitenansichten unterschiedlicher Lagen der Visualisierungseinheit, die aus dem Kopf-Tracking und aus zwei nacheinander aktivierten Steuervorschriften für die Bewegung der Visualisierungseinheit resultieren, jeweils mit Bezug zu einem Kopf eines Patienten, gemäß der bevorzugten Ausführungsform aus Fig. 1;
- Fign. 3a bis 3d: Seitenansichten unterschiedlicher Lagen der Visualisierungseinheit, die aus dem Kopf-Tracking und aus zwei nacheinander aktivierten Steuervorschriften für die Bewegung der Visualisierungseinheit resultieren, jeweils mit Bezug zu einem Kopf eines Patienten, gemäß der bevorzugten Ausführungsform aus Fign. 1 und 2;
- Fig. 4: eine Operationsbrille mit einem im sichtbaren Spektrum vorgesehenen optischen Muster als Marker zur externen Erfassung durch eine Trackingkamera des Kopf-Trackingsystems, gemäß der bevorzugten Ausführungsform;
- Fig. 5: eine Operationsbrille mit Infrarot-Reflektormarkern zur externen Erfassung durch eine IR-Trackingkamera des Kopf-Trackingsystems, gemäß einer weiteren bevorzugten Ausführungsform;
- Fig. 6: ein Steuersystem mit einer aktivierten Steuervorschrift zur Bewegungssteuerung der Visualisierungseinheit in einer perspektivischen Ansicht, gemäß der bevorzugten Ausführungsform; und
- Fig. 7: ein Verfahren zur Bewegungssteuerung der robotergeführten Visualisierungseinheit, gemäß einer bevorzugten Ausführungsform.

Die Figuren sind schematischer Natur und sollen dem Verständnis der Erfindung dienen. Gleiche Elemente sind mit denselben Bezugszeichen versehen. Merkmale verschiedener Ausführungsformen können untereinander ausgetauscht werden.

### Detaillierte Beschreibung bevorzugter Ausführungsformen

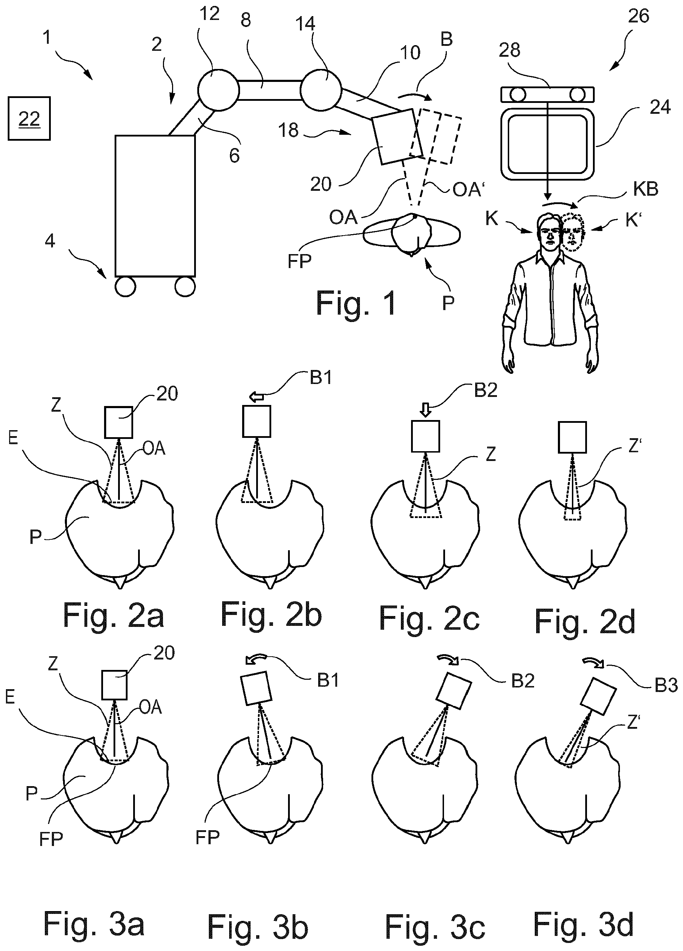

Figur 1 zeigt ein chirurgisches Steuersystem 1 gemäß einer bevorzugten Ausführungsform in schematischer Seitenansicht. Das Steuersystem 1 hat einen chirurgischen Roboter 2 mit einer Roboterbasis 4, die in der gezeigten Ausführungsform mobil ist, um den Roboter 2 bedarfsgerecht an verschiedenen Stellen eines Operationssaals im Krankenhaus einsetzen zu können. In der gezeigten Ausführungsform ist die mobile Basis 4 lagefest arretiert und bildet einen lokalen Bezugspunkt, an dem ein mehrsegmentierter Roboterarm mit mehreren Roboterarm-Segmenten 6, 8, 10 angebunden ist, welche über Gelenke 12, 14 jeweils miteinander verbunden sind. Alternativ kann die Roboterbasis natürlich fest sein. Auf diese Weise können die Roboterarm-Segmente 8 und 10 zueinander aktiv bewegt werden und der Roboterarm 6, 8, 10, 12, 14 lässt sich im Gesamten steuern.

An einer endständigen Seite des Endsegments 10 des Roboterarms ist eine in der gezeigten Ausführungsform als chirurgisches Operationsmikroskop ausgeführte Visualisierungseinheit 18, genauer gesagt deren Mikroskopkopf 20, befestigt, dessen optische Ache OA auf einen Kopf des Patienten P gerichtet ist.

Das Steuersystem 1 hat zudem ein Trackingsystem 26, das in der gezeigten Ausführungsform gemäß Figur 1 eine 3D-Trackingkamera 28 mit Sicht auf ein Gesicht O des Operateurs hat. Die Trackingkamera 28 verfolgt das Gesicht und erfasst somit beispielsweise dessen Lage O.

Die Visualisierungseinheit 18 erstellt entlang ihrer optischen Achse OA fortwährend eine intrakorporale Aufnahme vom Patienten P und stellt diese einer Steuereinheit 22 des Steuersystems 1 digital bereit. Die Steuereinheit 22 steuert in Folge einen (externen) Monitor 24 eines Anzeigesystems des Steuersystems 1 an, auf welchem dann die Aufnahme dem Operateur angezeigt wird. Der Operateur ist dem Monitor 24 zugewandt (in Figur 1 zur Veranschaulichung des Gesichtes des Operateurs ist dies invertiert dargestellt) und betrachtet auf dem Monitor 24 die zeitaktuell angezeigte Aufnahme des Patienten P in einem entlang der optischen Achse OA ausgerichteten Sichtfeld der Visualisierungseinheit 18.

Erfindungsgemäß kann nun eine Bewegung B der robotergeführten Visualisierungseinheit 18, ohne die Benutzung der Hände des Operateurs, durch das Tracking des Kopfes K, K' des Operateurs, insbesondere durch das Tracking des Gesichtes des Operateurs, gesteuert werden.

Zum einen ist die Trackingkamera 28 mit der Steuereinheit 22 signalverbunden und stellt dieser kontinuierlich eine Aufnahme des Gesichtes digital bereit.

Des Weiteren ist in der Steuereinheit 22 ein Algorithmus zum maschinellen Sehen zur Ausführung gespeichert, durch den die Steuereinheit 22 eine Lage des Kopfes K, K' auf Basis der Aufnahme des Gesichtes bestimmt.

Erfindungsgemäß ist in der Steuereinheit 22 zudem eine Steuervorschrift zur Ausführung gespeichert, mittels der die Steuereinheit 22 aus der erfassten Kopfbewegung KB des Operateurs von K zu K' eine Bewegung B der Visualisierungseinheit 18 bestimmt und den Roboter mit einem dementsprechenden Steuersignal ansteuert, sodass der Roboter die Visualisierungseinheit 18 mit der Bewegung B führt und die optische Achse von OA auf OA' verstellt wird.

Gemäß dem in Figur 1 gezeigten Ausführungsbeispiel ist somit eine Steuervorschrift zur Ausführung aktiviert, die bei jeglicher Kopfbewegung KB von K zu K' einen Fokuspunkt FP am Patienten P - und damit auch in der Aufnahme - fix hält und die Bewegung B der Visualisierungseinheit 18 auf einen Orbit des Fokuspunktes FP beschränkt. Dementsprechend führt die in Figur 1 dargestellte Kopfbewegung KB von links nach rechts (K zu K') bei gleichbleibendem Abstand der Visualisierungseinheit 18 zum Fokuspunkt FP zu einer Verschwenkung der Visualisierungseinheit 18 um den Fokuspunkt FP. In anderen Worten: Keine Kopfbewegung des Operateurs führt zu einer Änderung des Fokuspunktes FP oder zu einer Änderung des Abstandes zum Fokuspunkt. Der Fokuspunkt am Gewebe des Patienten kann also mit konstantem Abstand aus verschiedenen Blickwinkeln betrachtet werden, indem der Operateur eine Verschiebung oder eine Neigung des Kopfes vornimmt, nach links, rechts, oben oder unten.

Die Figuren 2a bis 2d zeigen die sequentielle Anwendung von in der Steuereinheit 22 zur Ausführung gespeicherten Steuervorschriften durch aufeinanderfolgende Aktivierung und Deaktivierung, bzw. durch einen Wechsel der aktiven Steuervorschrift, durch den Operateur.

In Figur 2a ist eine Ausgangslage dargestellt. Der Kopf des Operateurs hat demgemäß eine Lage K (nicht dargestellt), die Visualisierungseinheit 18 hat eine Lage mit einer optischen Achse OA, einem Sichtfeld oder Zoom Z und einer Bildebene E.

Gemäß Figur 2b erfolgt die Aktivierung einer Steuervorschrift, die alle translatorischen Bewegungskomponenten einer getrackten Kopfbewegung KB (nicht dargestellt) in translatorische Bewegungskomponenten der Bewegung B der Visualisierungseinheit 18 transformiert.

Gemäß Figur 2b erfolgt eine Bewegung des Kopfes nach links. In Folge bestimmt die Steuereinheit 22 auf Basis der erfassten Kopfbewegung und der genannten Steuervorschrift eine Bewegung B1 der Visualisierungseinheit 18 nach links mit Bezug zum Patienten P. Die Bildebene E und der Zoom Z bleiben konstant.

Die genannte Steuervorschrift ist weiterhin aktiv.

Gemäß Figur 2c erfolgt nun eine Bewegung des Kopfes nach vorne (nicht dargestellt). In Folge bestimmt die Steuereinheit 22 auf Basis der erfassten Kopfbewegung und der genannten Steuervorschrift eine Bewegung B2 der Visualisierungseinheit 18 nach vorne mit Bezug zum Patienten P, das heißt hin zu dem Patienten P. Die Bildebene E und der Zoom Z bleiben immer noch konstant.

Die bis hierhin aktivierte Steuervorschrift wird nun deaktiviert und der Operateur will bei - mit Bezug zu Figur 2c - gleichbleibender optischer Achse OA den Zoom verstärken.

Der Operateur aktiviert hierzu eine Steuervorschrift, die es ihm erlaubt, mit einer in und entgegen seiner Sichtrichtung gerichteten Kopfbewegung eine innere Verstellung einer Optik der Visualisierungseinheit 18 so zu bewirken, dass er mittels der Kopfbewegung in die Aufnahme hinein- und hinauszoomen kann.

In Folge bewegt er den Kopf hin zu dem Monitor 24, also hin zu der Trackingkamera 28. Die Trackingkamera 28 erfasst diese Bewegung und die Steuereinheit 22 erkennt die Bewegung des Kopfes nach vorne anhand der Analyse des Gesichtes des Operateurs mit Hilfe des maschinellen Sehens. Mittels der aktiven Steuervorschrift steuert die Steuereinheit 22 die Visualisierungseinheit 18 an, den Zoom von Z gemäß Figur 2c auf Z' gemäß Figur 2d zu verstärken.

In Figur 3a ist wieder die Ausgangslage gemäß Figur 2a dargestellt.

Durch den Operateur erfolgt die Aktivierung der Steuervorschrift, die bereits anhand Figur 1 beschrieben wurde, die also bei rotatorischen Kopfbewegungen des Operateurs den Fokuspunkt FP am Patienten P - und damit auch in der Aufnahme - fix hält und die Bewegung B der Visualisierungseinheit 18 auf einen Orbit des Fokuspunktes FP beschränkt (vgl. Figur 1).

Dementsprechend resultiert gemäß Figur 3b aus einer Rotation des Kopfes nach links eine orbitale Rotationsbewegung B1 der Visualisierungseinheit 18 um den Fokuspunkt FP nach links, sowie gemäß Figur 3c aus einer Rotation des Kopfes nach rechts eine orbitale Rotationsbewegung B2 der Visualisierungseinheit 18 um den Fokuspunkt FP nach rechts.

Alternativ erfolgt im Anschluss an die Ausgangslage gemäß Figur 3a die Aktivierung einer Steuervorschrift, die es ihm ergänzend zur letztgenannten Steuervorschrift erlaubt, mittels einer Kopfbewegung nach vorne und nach hinten, den Zoom zu verstellen.

Ausgehend von der Ausgangslage gemäß Figur 3a rotiert er nun den Kopf nach rechts und bewegt ihn gleichzeitig oder sequentiell hin zur Trackingkamera 28, also hin zu dem Monitor 24. Aufgrund der aktiven Steuervorschrift erfolgt dann sowohl die orbitale Rotationsbewegung B3 der Visualisierungseinheit 18 um den Fokuspunkt FP nach rechts als auch die interne Verstellung des Zooms von Z auf Z'

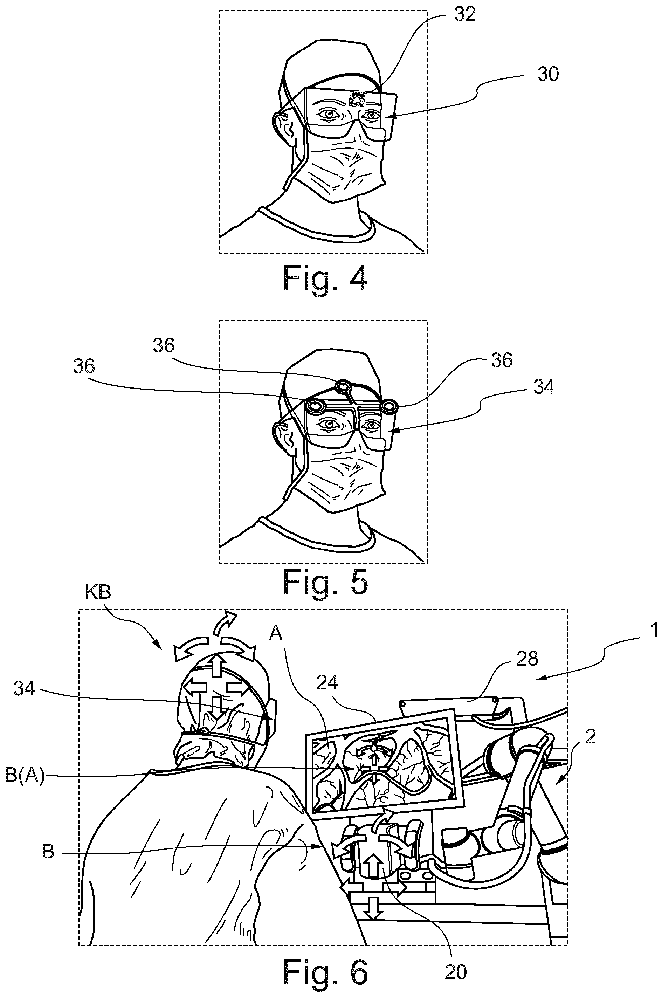

Die Figuren 4 und 5 zeigen jeweils eine als OP-Brille ausgestaltete, mobile Tragevorrichtung 30, 34 des Trackingsystems 26, an der jeweils ein geometrisches Referenzsystem 32, 36 als externer Marker vorgesehen ist, wobei es sich in Figur 4 um einen zentral im Bereich der Nasenwurzel angeordneten Matrixcode, ähnlich einem QR-Code, handelt und in Figur 5 um drei IR-Reflektormarker, von denen einer an einem Ausleger, zentral oberhalb der Nasenwurzel angeordnet ist und die beiden anderen, in zur Symmetrieebene der OP-Brille 34 spiegelsymmetrischer Anordnung, in linker und rechter oberer Ecke der OP-Brille 34 angeordnet sind.

Die Figur 6 zeigt das Steuersystem 1 mit der Trackingkamera 28, dem Roboter 2, dem am Roboterarm-Endsegment endständig befestigten Mikroskopkopf 20 der Visualisierungseinheit 18, dem Operateur mit der als OP-Brille 34 ausgestalteten Tragvorrichtung mit IR-Reflektorkörpern und den Monitor 24 des Anzeigesystems mit der darauf angezeigten Aufnahme A. Dabei ist zu erkennen, dass eine Steuervorschrift aktiv ist, die alle drei Rotationen (rx, ry, rz) des Kopfes aber nur zwei (x, y) von drei möglichen Translationen der Kopfbewegung KB, die parallel zur Bildebene E erfolgen, auf die Bewegung B des Mikroskopkopfes 20 überträgt.

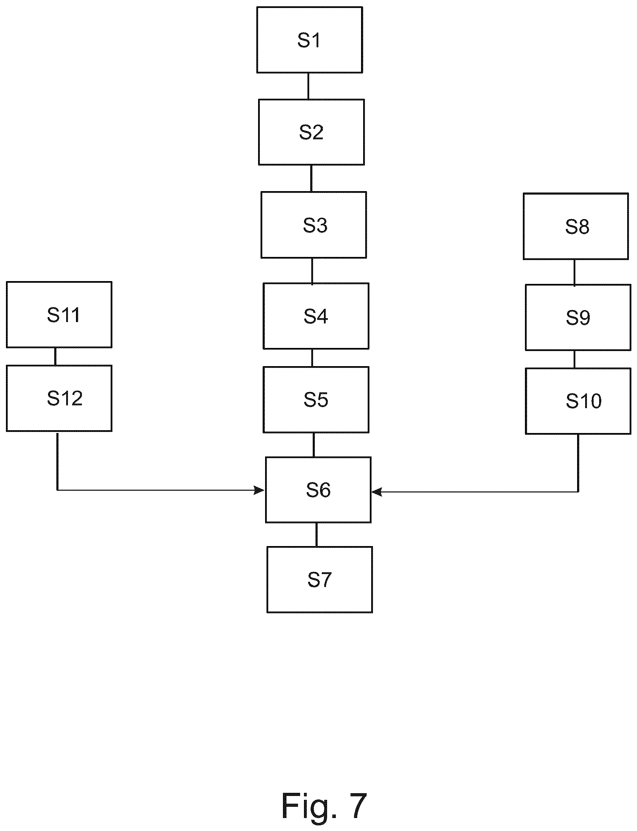

Die Figur 7 zeigt ein computerimplementiertes Verfahren zum Steuern einer Bewegung einer robotergeführten Visualisierungseinheit 18, insbesondere von deren Visualisierungskopf 20, für das chirurgische Steuersystem 1 gemäß der vorangegangenen Beschreibung. Dabei erfolgen zunächst Schritte:
Erstellen S1 und Bereitstellen S2 einer zeitaktuellen Aufnahme A eines Eingriffsgebietes des Patienten P, durch die Visualisierungseinheit 20; und
Anzeigen S3 der bereitgestellten Aufnahme A, durch das Anzeigesystem, insbesondere den Monitor 24.

Es folgen offenbarungsgemäß Schritte:
Erfassen S4 einer Kopfbewegung KB eines Operateurs durch ein Kopf-Trackingsystem 26;
Aufgliedern S5 der erfassten Kopfbewegung KB als ein Eingangssignal in translatorische und/oder rotatorische Bewegungskomponenten in oder um Achsen eines Koordinatensystems, insbesondere eines kartesischen Koordinatensystems, durch eine Steuereinheit 22;
Bestimmen S6 eines Steuersignals für eine robotergeführte Bewegung B der Visualisierungseinheit 20 auf Basis der Bewegungskomponenten der Kopfbewegung KB unter Verwendung wenigstens einer in der Steuereinheit 22 zur Ausführung gespeicherten Steuervorschrift; und
Ansteuern S7 des Roboters 2 mit dem Steuersignal, sodass die robotergeführte Bewegung B der Visualisierungseinheit 20 auf Basis der Kopfbewegung KB und der Steuervorschrift erfolgt.

Gemäß einer bevorzugten Ausführung des Verfahrens hat es vorzugsweise Schritte:
Erfassen S8 einer Anforderung zur Aktivierung der wenigstens einen Steuervorschrift, durch ein mit der Steuereinheit 22 signalverbundenes Anforderungssystem;
Ansteuern S9 der Steuereinheit 22, die wenigstens eine, insbesondere gültige, Steuervorschrift zu aktivieren, durch das Anforderungssystem, und
Aktivieren S10 der wenigstens einen, insbesondere gültigen, Steuervorschrift, durch die Steuereinheit 22.

Vorzugsweise ist die Steuervorschrift solange aktiviert ist, bis durch das Anforderungssystem eine Anforderung zur Deaktivierung erfasst wird oder bis von der Steuereinheit ein Deaktivierungskriterium als erfüllt bestimmt wird. Demgemäß hat eine Weiterbildung des Verfahrens Schritte:
Erfassen S11 einer Anforderung zur Deaktivierung der wenigstens einen Steuervorschrift, durch das Anforderungssystem, oder
Bestimmen eines Deaktivierungskriteriums als erfüllt, durch die Steuereinheit,
und in Abhängigkeit der erfassten Anforderung zur Deaktivierung oder des als erfüllt bestimmten Deaktivierungskriteriums:
   Deaktivieren S12 der Steuervorschrift durch die Steuereinheit 22.

Um dem Operateur die Möglichkeit zu geben, auf unterschiedliche Steuervorschriften zugreifen zu können, die bei gleicher Kopfbewegung in unterschiedlicher Bewegung B der Visualisierungseinheit 18 resultieren, weist eine Weiterbildung des Verfahrens wenigstens einen der folgenden Schritte auf:
Auswahl einer von mehreren in der Steuereinheit 22 zur Ausführung gespeicherten Steuervorschriften als gültige Steuervorschrift, durch eine Auswahleinheit, die dafür angepasst ist, eine aus mehreren Steuervorschriften als gültige auszuwählen und/oder eine aktuell gültige der Steuervorschriften zu wechseln, oder
Bestimmen einer von mehreren Steuervorschriften als gültige Steuervorschrift, durch die Steuereinheit 22.

### Bezugszeichenliste

- 1: chirurgisches Steuersystem
- 2: Roboter
- 4: Roboterbasis
- 6, 8, 10: Roboterarm-Segment
- 12, 14: Gelenk
- 16: Endabschnitt Roboterarm
- 18: Visualisierungseinheit
- 20: Visualisierungskopf
- 22: Steuereinheit
- 24: Monitor
- 26: Trackingsystem
- 28: Trackingkamera
- 30: Tragvorrichtung
- 32: optischer Marker
- 34: Tragvorrichtung
- 36: IR-Marker

- P: Patient
- A: Aufnahme
- E: Bildebene Aufnahme
- OA: optische Achse Visualisierungseinheit
- FP: Fokuspunkt
- Z: Zoom
- B: Bewegung Visualisierungseinheit
- K: Kopf Operateur
- KB: Kopfbewegung

- S1: Schritt Erstellen Aufnahme
- S2: Schritt Bereitstellen Aufnahme
- S3: Schritt Anzeigen Aufnahme
- S4: Schritt Erfassen Kopfbewegung
- S5: Schritt Aufgliedern Bewegungskomponenten
- S6: Schritt Bestimmen Steuersignal
- S7: Schritt Ansteuern Roboter
- S8: Erfassen Anforderung Aktivieren
- S9: Ansteuern Steuereinheit
- S10: Aktivieren Steuervorschrift
- S11: Erfassen Anforderung Deaktivieren
- S12: Deaktivieren Steuervorschrift

## Patentansprüche

1. Chirurgisches Steuersystem (1) zur Steuerung einer robotergeführten Visualisierungseinheit (18) bei einem chirurgischen Eingriff, mit:
einem Roboter (2) mit einer Roboterbasis (4) als lokalen Anbindungspunkt des Roboters (2) und einem an die Roboterbasis (4) angebundenen, bewegbaren Roboterarm mit einer Vielzahl an Roboterarm-Segmenten (6, 8, 10), welche mittels Gelenken miteinander verbunden sind;
der Visualisierungseinheit (18), die an dem Roboterarm angebunden ist und dafür angepasst ist, eine Aufnahme (A) eines Eingriffsgebietes zu erstellen und bereitzustellen;
ein Kopf-Trackingsystem (26), das dafür angepasst ist, eine Kopfbewegung (KB) eines Operateurs zu erfassen;
eine Steuereinheit (22), die dafür angepasst ist, den Roboter (2) mit einem in Abhängigkeit der erfassten Kopfbewegung (KB) bestimmten Steuersignal anzusteuern, sodass die robotergeführte Bewegung (B) der Visualisierungseinheit (18) in Abhängigkeit der erfassten Kopfbewegung (KB) erfolgt; und
eine mit der Steuereinheit (22) signalverbundene Aktivierungseinrichtung, die dafür angepasst ist, eine Steuerung der robotergeführten Bewegung (B) der Visualisierungseinheit (18) zu aktivieren oder zu deaktivieren, wobei die Aktivierungseinrichtung als Totmannschalter ausgebildet ist, sodass die Steuerung der robotergeführten Bewegung (B) der Visualisierungseinheit (18) nur bei betätigter Aktivierungseinrichtung möglich ist.

2. Chirurgisches Steuersystem (1) nach Anspruch 1, wobei eine visuelle Anzeigevorrichtung (24) zur Anzeige der mit der Visualisierungseinheit (18) erstellten und bereitgestellten Aufnahme (A) des Eingriffsgebietes bereitgestellt ist, wobei die visuelle Anzeigevorrichtung (24) als ein externer Monitor (24) ausgestaltet ist.

3. Chirurgisches Steuersystem (1) nach Anspruch 1 oder 2, wobei das Kopf-Trackingsystem (26) aufweist:
wenigstens einen am Kopf befestigbaren Marker (32; 36) und eine extern angeordnete optische Erfassungseinheit (28), die dafür angepasst ist, den wenigstens einen Marker (36) optisch zu erfassen.

4. Chirurgisches Steuersystem (1) nach Anspruch 2 und 3, wobei die optische Erfassungseinheit (28) am externen Monitor (24) angeordnet oder befestigt ist.

5. Chirurgisches Steuersystem (1) nach einem der Ansprüche 1 bis 4, wobei die Steuereinheit (22) dafür angepasst ist, die Visualisierungseinheit (18) mit einem in Abhängigkeit der erfassten Kopfbewegung (KB) bestimmten Verstellsignal anzusteuern, sodass eine innere Verstellung eines Bildausschnitts oder Zooms (Z) in Abhängigkeit der erfassten Kopfbewegung (KB) erfolgt.

6. Chirurgisches Steuersystem (1) nach einem der Ansprüche 1 bis 5, wobei die Steuereinheit (22) dafür angepasst ist, die Visualisierungseinheit (18) mit einem in Abhängigkeit der erfassten Kopfbewegung (KB) bestimmten Verstellsignal anzusteuern, sodass eine innere Verstellung eines Fokus oder Brennpunktes der Visualisierungseinheit (18) erfolgt.

7. Chirurgisches Steuersystem (1) nach einem der Ansprüche 1 bis 6, wobei die Steuereinheit (22) dafür angepasst ist, die erfasste Kopfbewegung (KB) in Bewegungskomponenten in oder um Achsen eines Koordinatensystems aufzugliedern, und vorzugsweise auf die Bewegungskomponenten wenigstens eine in der Steuereinheit (22) gespeicherte Steuervorschrift anzuwenden, um das Steuersignal zu bestimmen, und/oder wenigstens eine in der Steuereinheit (22) gespeicherte Verstellvorschrift anzuwenden, um das Verstellsignal zu bestimmen.

8. Chirurgisches Steuersystem (1) nach Anspruch 7, wobei die wenigstens eine Steuervorschrift das Steuersignal derart bestimmt:
dass die robotergeführte Bewegung (B) der Visualisierungseinheit (18) auf eine orbitale, vorzugsweise sphärische, Bewegung beschränkt ist, wobei vorzugsweise ein Fokuspunkt (FP) der Aufnahme (A) fix ist und die Bewegung (B) auf einen Orbit, vorzugsweise eine Sphäre, des Fokuspunktes (FP) beschränkt ist.

9. Chirurgisches Steuersystem (1) nach Anspruch 7 oder 8, wobei die Steuereinheit (22) dafür angepasst ist, das jeweilige Verstellsignal so zu bestimmen, dass die Kopfbewegung (KB), die in Sichtrichtung des Operateurs gerichtet ist, ein Heranzoomen bewirkt oder eine Verlagerung des Fokus in Blickrichtung bewirkt, und dass die Kopfbewegung (KB), die entgegen der Sichtrichtung des Operateurs gerichtet ist, ein Herauszoomen bewirkt oder eine Verlagerung des Fokus entgegen der Blickrichtung bewirkt.

10. Chirurgisches Steuersystem (1) nach einem der Ansprüche 1 bis 9, wobei die Aktivierungseinrichtung dafür angepasst ist, eine Steuerung der inneren Verstellung des Bildausschnitts oder Zooms (Z) der Visualisierungseinheit (18) und/oder eine Steuerung der inneren Verstellung des Fokus oder Brennpunktes der Visualisierungseinheit (18) zu aktivieren oder zu deaktivieren.

11. Chirurgisches Steuersystem (1) nach einem der Ansprüche 1 bis 10, wobei die Aktivierungseinrichtung als ein Fußpedal oder ein Mundschalter ausgestaltet ist.

12. Chirurgisches Steuersystem (1) nach einem der Ansprüche 1 bis 11, wobei das Kopf-Trackingsystem (26) aufweist:
wenigstens eine optische Erfassungseinheit, die dafür angepasst ist, die Kopfbewegung (KB) mittels maschinellem Sehen des Gesichtes des Operateurs zu erfassen.

13. Chirurgisches Steuersystem (1) nach einem der Ansprüche 1 bis 12, wobei das Kopf-Trackingsystem (26) ergänzend aufweist:
wenigstens eine am Kopf befestigbare Beschleunigungs- oder Trägheitssensoreinheit, die dafür angepasst ist, die Kopfbewegung (KB) zu erfassen.

14. Computerimplementiertes Verfahren zum Steuern einer Bewegung einer robotergeführten Visualisierungseinheit (18) bei einem chirurgischen, insbesondere neurochirugischen, Eingriff, mit einem chirurgischen Steuersystem (1) gemäß einem der Ansprüche 1 bis 13, wobei die Visualisierungseinheit (18) ein chirurgisches Mikroskop oder ein Exoskop oder eine chirurgische Ultraschallsonde ist, mit Schritten:
Erstellen (S1) und Bereitstellen (S2) einer zeitaktuellen Aufnahme (A) eines Eingriffsgebietes, durch die Visualisierungseinheit (18); und
Anzeigen (S3) der bereitgestellten Aufnahme (A), durch eine visuelle Anzeigevorrichtung (24);
und die Schritte aufweist:
Erfassen (S4) einer Kopfbewegung (KB) eines Operateurs durch ein Kopf-Trackingsystem (26);
Bestimmen (S6) eines Steuersignals für eine robotergeführte Bewegung (B) der Visualisierungseinheit (18) in Abhängigkeit der erfassten Kopfbewegung (KB), durch eine Steuereinheit (22); und
Ansteuern (S7) des Roboters (2) mit dem Steuersignal, sodass die robotergeführte Bewegung (B) der Visualisierungseinheit (18) in Abhängigkeit der erfassten Kopfbewegung (KB) erfolgt, durch die Steuereinheit (22).

15. Computerlesbares Speichermedium und/oder Computerprogramm, das Befehle umfasst, die bei einer Ausführung durch einen Computer diesen veranlassen, die Verfahrensschritte des Verfahrens zum Steuern einer Visualisierungseinheit (18) gemäß Anspruch 14 auszuführen.

## Claims

1. A surgical control system (1) for controlling a robot-guided visualization unit (18) during a surgical procedure, comprising:
a robot (2) including a robot base (4) as a local connection point of the robot (2) and a movable robot arm connected to the robot base (4) including a plurality of robot arm segments (6, 8, 10), which are connected to each other via joints;
the visualization unit (18), which is connected to the robot arm and is configured to create and provide an image (A) of a surgical area;
a head tracking system (26) configured to detect head motion (KB) of a surgeon;
a control unit (22) configured to control the robot (2) with a control signal determined in dependence on the detected head motion (KB), so that the robot-guided motion (B) of the visualization unit (18) takes place in dependence on the detected head motion (KB), and
an activation device connected to the control unit (22) by signal, which is configured to activate or deactivate a control of the robot-guided motion (B) of the visualization unit (18), wherein the activation device is designed as a dead man's switch, so that control of the robot-guided motion (B) of the visualization unit (18) is only possible when the activation device is actuated.

2. The surgical control system (1) according to claim 1, wherein a visual display device (24) is provided for displaying the image (A) of the surgical area created and provided by the visualization unit (18), the visual display device (24) being designed as an external monitor (24).

3. The surgical control system (1) according to claim 1 or 2, wherein the head tracking system (26) comprises:
at least one marker (32; 36) that can be attached to the head, and an externally arranged optical detection unit (28) that is configured to optically detect the at least one marker (36).

4. The surgical control system (1) according to claims 2 and 3, wherein the optical detection unit (28) is arranged on or attached to the external monitor (24).

5. The surgical control system (1) according to any of claims 1 to 4, wherein the control unit (22) is configured to control the visualization unit (18) by an adjustment signal determined depending on the detected head motion (KB), so that an internal adjustment of an image portion or zoom (Z) takes place as a function of the detected head motion (KB).

6. The surgical control system (1) according to any of claims 1 to 5, wherein the control unit (22) is configured to control the visualization unit (18) by an adjustment signal determined depending on the detected head motion (KB), so that an internal adjustment of a focus or focus point of the visualization unit (18) takes place.

7. The surgical control system (1) according to any of claims 1 to 6, wherein the control unit (22) is configured to break down the detected head motion (KB) into motion components in or around axes of a coordinate system, and preferably apply at least one control rule stored in the control unit (22) to the motion components in order to determine the control signal, and/or to apply at least one adjustment rule stored in the control unit (22) in order to determine the adjustment signal.

8. The surgical control system (1) according to claim 7, wherein the at least one control rule determines the control signal in such a way
that the robot-guided motion (B) of the visualization unit (18) is limited to an orbital, preferably spherical, motion, wherein preferably a focus point (FP) of the image (A) is fixed and the motion (B) is limited to an orbit, preferably a sphere, of the focus point (FP).

9. The surgical control system (1) according to claim 7 or 8, wherein the control unit (22) is configured to determine the respective adjustment signal such that the head motion (KB) directed in the surgeon's line of view causes zooming in or a shift in focus in the line of gaze, and that the head motion (KB) directed against the surgeon's line of view causes zooming out or a shift in focus against the line of gaze.

10. The surgical control system (1) according to any of claims 1 to 9, wherein the activation device is configured to activate or deactivate a control of the internal adjustment of the image portion or zoom (Z) of the visualization unit (18) and/or a control of the internal adjustment of the focus or focus point of the visualization unit (18).

11. The surgical control system (1) according to one of claims 1 to 10, wherein the activation device is designed as a foot pedal or a mouth switch.

12. The surgical control system (1) according to any of claims 1 to 11, wherein the head tracking system (26) comprises:
at least one optical detection unit configured to detect the head motion (KB) by means of machine vision of the surgeon's face.

13. The surgical control system (1) according to any of claims 1 to 12, wherein the head tracking system (26) additionally comprises:
at least one acceleration or inertia sensor unit that can be attached to the head and is configured to detect head motion (KB).

14. A computer-implemented method for controlling the motion of a robot-guided visualization unit (18) during a surgical, in particular neurosurgical, procedure, with a surgical control system (1) according to claims 1 to 13, wherein the visualization unit (18) is a surgical microscope, or an exoscope, or a surgical ultrasonic probe, comprising the steps of:
creating (S1) and providing (S2) a real-time image (A) of a surgical area by the visualization unit (18); and
displaying (S3) the provided image (A) by a visual display device (24);
and the steps of
detecting (S4) a head motion (KB) of a surgeon by a head tracking system (26);
determining (S6) a control signal for a robot-guided motion (B) of the visualization unit (18) as a function of the detected head motion (KB) by a control unit (22); and
controlling (S7) the robot (2) by the control signal so that the robot-guided motion (B) of the visualization unit (18) is performed as a function of the detected head motion (KB), by the control unit (22).

15. The computer-readable storage medium and/or computer program comprising commands which, when executed by a computer, cause the computer to perform the steps of the method for controlling a visualization unit (18) according to claim 14.

## Revendications

1. Système de commande chirurgical (1) pour la commande d'une unité de visualisation robotisée (18) lors d'une intervention chirurgicale, doté de :
un robot (2) avec une base de robot (4) comme point de liaison local du robot (2) et un bras de robot mobile lié à la base de robot (4) avec une pluralité de segments de bras de robot (6, 8, 10), qui sont reliés entre eux au moyen d'articulations ;
l'unité de visualisation (18) qui est liée au bras de robot et est adaptée pour réaliser et fournir une prise de vue (A) d'une zone d'intervention ;
un système de suivi de tête (26) qui est adapté pour détecter un mouvement de tête (KB) d'un opérateur ;
un dispositif de commande (22) qui est adapté pour commander le robot (2) avec un signal de commande déterminé en fonction du mouvement de tête (KB) détecté, de sorte que le mouvement robotisé (B) de l'unité de visualisation (18) s'effectue en fonction du mouvement de tête (KB) détecté ; et
un système d'activation lié par signal au dispositif de commande (22) qui est adapté pour activer ou désactiver une commande du mouvement robotisé (B) de l'unité de visualisation (18), dans lequel le système d'activation est configuré sous la forme d'un dispositif homme-mort, de sorte que la commande du mouvement robotisé (B) de l'unité de visualisation (18) n'est possible que lorsque le système d'activation est actionné.

2. Système de commande chirurgical (1) selon la revendication 1, dans lequel un dispositif d'affichage visuel (24) est fourni pour l'affichage de la prise de vue (A), réalisée et fournie avec l'unité de visualisation (18), de la zone d'intervention, dans lequel le dispositif d'affichage visuel (24) est conçu comme un moniteur externe (24).

3. Système de commande chirurgical (1) selon la revendication 1 ou 2, dans lequel le système de suivi de tête (26) présente au moins un marqueur (32 ; 36) pouvant être fixé sur la tête et une unité de détection optique (28) agencée en externe qui est adaptée pour détecter optiquement l'au moins un marqueur (36).

4. Système de commande chirurgical (1) selon les revendications 2 et 3, dans lequel l'unité de détection optique (28) est agencée ou fixée sur un moniteur externe (24).

5. Système de commande chirurgical (1) selon l'une quelconque des revendications 1 à 4, dans lequel le dispositif de commande (22) est adapté pour commander l'unité de visualisation (18) au moyen d'un signal de réglage déterminé en fonction du mouvement de tête (KB) détecté, de sorte qu'un réglage interne d'un extrait d'image ou d'un zoom (Z) est effectué en fonction du mouvement de tête (KB) détecté.

6. Système de commande chirurgical (1) selon l'une quelconque des revendications 1 à 5, dans lequel le dispositif de commande (22) est adapté pour commander l'unité de visualisation (18) au moyen d'un signal de réglage déterminé en fonction du mouvement de tête (KB) détecté, de sorte qu'un réglage interne d'un foyer ou point focal de l'unité de visualisation (18) est effectué.

7. Système de commande chirurgical (1) selon l'une quelconque des revendications 1 à 6, dans lequel le dispositif de commande (22) est adapté pour décomposer le mouvement de tête (KB) détecté en composantes de mouvement dans ou autour d'axes d'un système de coordonnées, et de préférence pour appliquer aux composantes de mouvement au moins une consigne de commande enregistrée dans le dispositif de commande (22) afin de déterminer le signal de commande et/ou appliquer au moins une consigne de réglage enregistrée dans le dispositif de commande (22) afin de déterminer le signal de réglage.

8. Système de commande chirurgical (1) selon la revendication 7, dans lequel la au moins une consigne de commande détermine le signal de commande de telle sorte que :
le mouvement robotisé (B) de l'unité de visualisation (18) est limité à un mouvement orbital, de préférence sphérique, dans lequel un point focal (FP) de la prise de vue (A) est de préférence fixe, et le mouvement (B) est limité à une orbite, de préférence une sphère, du point focal (FP).

9. Système de commande chirurgical (1) selon la revendication 7 ou 8, dans lequel le dispositif de commande (22) est adapté pour déterminer le signal de réglage respectif, de telle manière que le mouvement de tête (KB) qui est dirigé dans le champ de vision de l'opérateur, provoque un zoom avant ou provoque un déplacement du foyer dans la direction du regard, et que le mouvement de tête (KB), qui est dirigé dans la direction opposée au champ de vision de l'opérateur, provoque un zoom arrière ou provoque un déplacement du foyer dans la direction opposée à la direction du regard.

10. Système de commande chirurgical (1) selon l'une quelconque des revendications 1 à 9, dans lequel le système d'activation est adapté pour activer ou désactiver une commande du réglage interne de l'extrait d'image ou du zoom (Z) de l'unité de visualisation (18) et/ou une commande du réglage interne du foyer ou point focal de l'unité de visualisation (18).

11. Système de commande chirurgical (1) selon l'une quelconque des revendications 1 à 10, dans lequel le système d'activation est conçu comme une pédale ou un contacteur buccal.

12. Système de commande chirurgical (1) selon l'une quelconque des revendications 1 à 11, dans lequel le système de suivi de tête (26) présente :
au moins une unité de détection optique qui est adaptée pour détecter le mouvement de tête (KB) au moyen de la vision par ordinateur du visage de l'opérateur.

13. Système de commande chirurgical (1) selon l'une quelconque des revendications 1 à 12, dans lequel le système de suivi de tête (26) présente en complément :
au moins une unité de capteur d'accélération ou d'inertie pouvant être fixée sur la tête qui est adaptée pour détecter le mouvement de tête (KB).

14. Procédé mis en œuvre par ordinateur pour commander un mouvement d'une unité de visualisation (18) robotisée lors d'une intervention chirurgicale, en particulier neurochirurgicale, doté d'un système de commande chirurgical (1) selon l'une quelconque des revendications 1 à 13, dans lequel l'unité de visualisation (18) est un microscope chirurgical ou un exoscope ou une sonde chirurgicale à ultrasons, comprenant les étapes consistant à :
réaliser (S1) et fournir (S2) par l'unité de visualisation (18) une prise de vue (A) en temps réel d'une zone d'intervention ; et
afficher (S3) par un dispositif d'affichage (24) la prise de vue (A) fournie ;
et comprenant les étapes consistant à :
détecter (S4) par un système de suivi de tête (26) un mouvement de tête (KB) d'un opérateur ;
déterminer (S6) par une unité de commande (22) un signal de commande pour un mouvement robotisé (B) de l'unité de visualisation (18) en fonction du mouvement de tête (KB) détecté ; et
commander (S7) par l'unité de commande (22) le robot (2) avec le signal de commande, de sorte que le mouvement robotisé (B) de l'unité de visualisation (18) s'effectue en fonction du mouvement de tête (KB) détecté.

15. Support de stockage lisible par ordinateur et/ou programme informatique comprenant des instructions qui, lorsqu'elles sont exécutées par un ordinateur, amènent celui-ci à exécuter les étapes de procédé du procédé de commande d'une unité de visualisation (18) selon la revendication 14.
